# EUROPEAN PATENT APPLICATION

(11) **EP 1 642 602 A2**
(43) Date of publication of application: **05.04.2006**
(21) Application number: 05255986.1
(22) Date of filing: 26.09.2005
(51) Int. Cl.: A61L 27/54, A61L 27/58

(54) **Bone void filler**

(30) Priority: 27.09.2004 US 950775
(71) Applicant: ETHICON, INC., Somerville, New Jersey 08876 (US)
(72) Inventor: Story, Brooks J., Franklin, MA 02038 (US); Schachter, Deborah, Edison, NJ 08817 (US); Martellota, Tracy, Franklin, MA 02038 (US)
(74) Representative: Fisher, Adrian John

(57) **Abstract**

Novel biodegradable bone void filler compositions. The bone void filler compositions have a biodegradable material component, an osteoconductive component, and a therapeutic agent. The bone void filler optionally contains an osteoinductive component. Also disclosed is a method of using the bone void filler compositions to fill a bone void.

## Description

### Technical Field

The field of art to which this invention relates is orthopedic medicine, more particularly, osteoconductive bone void fillers with therapeutic agents, and surgical procedures using these bone void fillers.

### Background of the Invention

Many orthopedic reconstructive surgical procedures require drilling or cutting into bone in order to harvest autologous implants used in the procedures or to create openings for the insertion of implants. In either case voids are created in bones. For example, it is known in anterior cruciate ligament (ACL) reconstructive procedures to use a bone-tendon-bone graft to reconstruct the knee. The bone-tendon-bone (BTB) graft is harvested from the patellar tendon, and has attached bone blocks at either end. One bone block is harvested from the patella, while at the opposite end of the graft a bone block is harvested from the tibia. The graft is then mounted in the knee in a conventional manner by drilling tunnels in the femur and the tibia, and then mounting one bone block in the tibial tunnel, and one bone block in the femoral tunnel, thereby completing the ACL reconstruction.

There are several deficiencies that may be associated with the presence of a void in a bone. The bone void may compromise the mechanical integrity of the bone, making the bone potentially susceptible to fracture until the void becomes ingrown with native bone. The bone void may also provide an opportunity for the incubation and proliferation of any infective agents that are introduced during the surgical procedure. Another common side effect of any surgery is ecchymosis in the surrounding tissue which results from bleeding of the traumatized tissues. Finally, the surgical trauma to the bone and surrounding tissues, such as the overlying periosteum, is known to be a significant source of postoperative pain and inflammation. In addition to the extreme discomfort, post-operative pain and inflammation severely limit the patient's range of motion, thereby delaying their return to function. The duration of the post-operative pain and inflammation can extend several days to weeks, however it is most intense in the first 3-7 days following surgery. It is known that the healing process is facilitated by an early return to limited motion thus, alleviation of pain and swelling will facilitate the post-operative healing process.

Post-operative pain in orthopedic surgery is typically treated with oral pain medications, which include acetaminophen, NSAIDs and opioid narcotics. These medications can have serious side effects including nausea, constipation, respiratory depression, dizziness, gastrointestinal distress, extreme drowsiness and resistance or addiction to the medications. The patient's functional abilities are impaired by opioids, making it difficult for the patient to return to normal activities. Post-operative pain also typically inhibits range of motion of the affected joint, thereby delaying the patient's physical therapy regimen. It has been demonstrated that early return to limited physical activity enhances the healing response. Thus, reduction of pain would have a positive effect on overall healing of the surgically repaired tissues.

External pumps connected to transcutaneous catheters have been used to dispense, post-operatively, a steady dose of anesthetic directly into a a surgical site. However, these pumps require full patient compliance to be effective. They also carry the risk of infection via the transcutaneous catheter. Moreover, because the anesthetic is delivered in a dilute aqueous form, the volume of the anesthetic solution can be substantial. This introduces practical pump size limitations that restrict their functionality to a period of 2-3 days.

As previously mentioned, BTB graft harvesting results in significant bone defects or voids in the patella and proximal tibia, resulting in compromised mechanical integrity. These harvest sites are sometimes filled by the surgeon with autologous bone chips that are generated during trimming of the bony ends of the graft to accommodate graft placement. It is postulated that these bone chips will encourage a faster rate of native bone growth into the void. However, the volume of these chips is typically not sufficient to completely fill the harvest sites, and it is not uncommon to leave the harvest site completely unfilled, relying on long term bone in-growth to fill the defect. Complete filling of the void by such bone in-growth can take up to two years, and incomplete filling of the void is common. A permanent, palpable indendation in the overlying skin can result from incomplete defect filling, along with associated pain during various activities, including kneeling, etc..

It is also known to use calcium phosphate or calcium sulfate cements in this art for filling bony defects. Such materials are also known to be used for long-term release of impregnated medications, such as antibiotics. However, such materials cure to a solid form, providing at best only trace levels of therapeutic agents released over a sustained period of time. These solid matrices cannot release the short-term higher doses of agents that are required to reduce post-operative pain and inflammation.

The use of gels and other polymeric based systems for local delivery of pain medication is also known and described in the literature. However, these systems are not osteoconductive and thus do not promote bone growth.

Accrodingly, there is a need in this art for compositions and surgical procedures that provide for immediate filling of a void in a bone, and that promote a rapid ingrowth of new native bone into the void, and which can also prevent or alleviate pain, inflammation and infection potentially resulting from a surgical procedure. There is a need for a system that can release high doses of a therapeutic agent within a short-term post-operative period, wherein the release system quickly erodes following this therapeutic agent release, leaving behind an osteoconductive matrix that can enhance the growth of native bone to fill the defect during the healing process.

### Summary of the Invention

Therefore novel biodegradable bone void filler compositions are disclosed. The bone void filler composition is comprised of a biodegradable material component, an osteoconductive component, and a therapeutic agent. The bone void filler optionally contains an osteoinductive component.

Yet another aspect of the present invention is a method of filling a bone void using the novel biodegradable bone void filler compositions of the present invention. A biodegradable bone void filler compostion is provided. The void filler has a biodegradable material component, an osteoconductive component, and a therapeutic agent. The composition optionally contains an osteoinductive component. The bone void filler composition is inserted or placed into a bone void such that the void is substantially filled.

These and other aspects and advantages of the present invention will become more apparent by the following description and accompanying drawings.

### Brief Description of the Drawings

FIG. 1 illustrates a knee undergoing a bone-tendon-bone graft ACL surgical reconstruction, illustrating the harvesting of a bone-tendon-bone graft and the resultant bone voids in the patella and the tibia.
FIG. 2 illustrates the knee of FIG. 1, wherein a bone void filler of the present invention has been placed in the bone voids.
FIG. 3 illustrates a schematic of the elution of pain medication from the void filler post-operatively.
FIG. 4 illustrates the void holes or harvest sites post-operatively wherein the voids are filled in with ingrown bone.

### Disclosure of Preferred Embodiment

The term biodegradable as used herein is defined to include materials that are degraded or broken down (chemically or physically) under physiological conditions in the body such that the degradation products are excretable or absorbable by the body.

The novel bone void filler compositions of the present invention, which are also used in the novel methods of the present invention, consist of a biodegradable material component, an osteoconductive component, and a therapeutic agent. The compositions optionally contain an osteoinductive component.

The biodegradable material component is made from biodegradable materials known in this art. The biodegradable material may be a polymer or co-polymer. Examples of polymers and co-polymers that can be used in the void fillers of the present invention include homopolymers, such as poly(glycolide), poly(lactide), poly(ε-caprolactone), poly(trimethylene carbonate), poly(para-dioxanone) and combinations thereof; copolymers, such as poly(lactide-co-glycolide), poly(epsilon-caprolactone-co-glycolide), poly(glycolide-co-trimethylene carbonate), and combinations thereof. The co-polymer may be statistically random co-polymers, segmented co-polymers, block co-polymers or graft copolymers.

Other biodegradable materials include albumin; casein; waxes such as fatty acid esters of glycerol, glycerol monosterate and glycerol disterate; starch, crosslinked starch; simple sugars such as glucose, ficoll, and polysucrose; polyvinyl alcohol; gelatine; modified celluloses such as carboxymethylcellulose (CMC), hydroxymethyl cellulose, hydroxyethyl cellulose (HEC), hydroxypropyl cellulose, hydroxypropyl-ethyl cellulose, hydroxypropyl-methyl cellulose (HPMC), sodium carboxymethyl cellulose, and cellulose acetate; sodium alginate; hyaluronic acid and derivatives; polyvinyl pyrollidone; polymaleic anhydride esters; polyortho esters; polyethyleneimine; glycols such as polyethylene glycol, methoxypolyethylene glycol, and ethoxypolyethylene glycol, polyethylene oxide; poly(1,3 bis(p-carboxyphenoxy) propane-co-sebacic anhydride; N,N-diethylaminoacetate; and block copolymers of polyoxyethylene and polyoxypropylene, combinations thereof, equivalents thereof and the like. It is particularly preferred to use a biodegradable polymer consisting of hydroxyethyl cellulose (HEC) or hyaluronic acid. The void filler compositions will contain a sufficient amount of biodegradable polymer to effectively allow release of an effective amount of therapeutic agent in the region surrounding the bone void. Typically the void filler compositions of the present invention will contain about 5 to about 99 weight percent of biodegradable material, more typically about 15 to about 75 weight percent, and preferably about 15 to about 55 weight percent. The void filler compositions will preferably allow the therapeutic agents contained therein to be released in a controlled manner over a period of time following surgery, e.g., about 3-7 days subsequent to surgery.

The osteoconductive component of the void filler compositions of the present invention contains a sufficiently effective amount of osteoconductive material to provide for bone in-growth into a void volume. The osteoconductive materials include, but are not limited to, alpha-tricalcium phosphate (alpha-TCP), beta-tricalcium phosphate (beta-TCP), calcium carbonate, barium carbonate, calcium sulfate, barium sulfate, hydroxyapatite, and mixtures thereof. In certain embodiments the osteoconductive material comprises a polymorph of calcium phosphate, equivalents thereof, combinations thereof and the like. A particularly preferred material is beta-tricalcium phosphate (beta-TCP). The amount of the osteoconductive material in the void filler compositions will typically range from about 5 to about 50 weight percent, more typically about 10 to about 40 weight percent, and preferably about 20 to about 30 weight percent. The amount of osteoconductive material in the void fillers of the present invention will be sufficient to effectively conduct bone growth into the void space.

The therapeutic agents of the bone void filler compositions of the present invention include pain medications such as, morphine, nonsteroidal anti-inflammatory drugs (NSAIDS), opioid analgesics (oxycodone, morphine, fentanyl, hydrocodone, naproxyphene, codeine, etc.), opioid/nonopioid combination analgesics (e.g. acetaminophen with codeine), acetaminophen, local anesthetics (benzocaine, lidocaine, procaine, bupivacaine, ropivacaine, mepivacaine, chloroprocaine, tetracaine, cocaine, etidocaine, prilocaine, procaine), alpha - 2 agonists (clonidine, xylazine, medetomidine, dexmedetomidine), VR1 antagonists, and combinations thereof and the like. If desired multiple therapeutic agents may be included having the same or different indications. Other types of therapeutic agents that may be incorporated into the bone void filler compositions include anti-infectives, such as antibiotics and antiviral agents; analgesics and analgesic combinations; anti-inflammatory agents; immunosupressives; steroids, including corticosteroids; naturally derived or genetically engineered proteins, polysaccharides, glycoproteins, or lipoproteins and the like. A sufficient amount of the therapeutic agent will be included in the void filler compositions of the present invention to be therapeutically effective. The amount will depend upon the type and nature of therapeutic agent and the characteristics of the patient as well as the nature of the surgical procedure.

The bone void filler compositions of the present invention may optionally contain an osteoinductive component to accelerate of ingrowth of bone into the osteoconductive component. Examples of osteoinductive materials suitable for use with the present invention include cell attachment mediators, such as peptide- containing variations of the "RGD" integrin binding sequence known to affect cellular attachment, biologically active ligands, and substances that enhance or exclude particular varieties of cellular or tissue ingrowth. Examples of such substances include integrin binding sequence, ligands, bone morphogenic proteins, epidermal growth factor, IGF-I, IGF-II, TGF-β I-III, growth differentiation factor, parathyroid hormone, vascular endothelial growth factor, hyaluronic acid, glycoprotein, lipoprotein, bFGF, TGF-β superfamily factors, BMP-2, BMP-4, BMP-6, BMP-12, BMP-14 (also known as CDMP (Cartilage Derived Morphogenic Protein) or GDF-5 (growth differentiation factor 5)), sonic hedgehog, GDF6, GDF8, PDGF, small molecules that affect the upregulation of specific growth factors, tenascin-C, fibronectin, thromboelastin, thrombin-derived peptides, heparin-binding domains, and the like.

The osteoinductive material may also comprise mineralized collagen particles mixed with a biologically derived substance selected from the group consisting of demineralized bone matrix (DBM), platelet rich plasma, bone marrow aspirate and bone fragments, all of which may be from autogenic, allogenic, or xenogenic sources.

A therapeutically effective amount of the osteoinductive material may be incorporated into the bone void filler compositions of the present invention. The amount of osteoinductive material in the void filler compositions of the present invention will be sufficient to effectively provide for accelerated bone in-growth into a void volume. The amount of osteoinductive material will typically be about 0.01 weight percent to about 1 weight percent.

The bone void filler compositions of the present invention may be used in a variety of physical states including fluids and solids and plastics. The embodiments of the fluid forms of the void filler compositions of the present invention may consist of viscous injectable liquids, moldable putties, caulk-like materials, gels, slurries combinations thereof and the like. The injectable fluid embodiments of the void fillers of the present invention will have sufficient viscosity at room temperature to be effectively flowable. The viscosity will typically range from about 50 centipoise to about 2,000,000 centipoise.

The solid embodiments may consist, for example, of pellets, tablets, molded or extruded structures, powders, plugs, capsules, granules, combinations thereof, and the like. The solid bone void filler compositions may be delivered into a void space in a variety of conventional manners. Granules or powders can be poured in tamped in place. Powders may be injected into the void using a suitable syringe and large gauge needle. Or a powder may be compressed into a tablet and placed into the bone void space. Alternately, a void filler composition formulation can be extruded into plugs that can be placed into the void space.

In one embodiment of the bone void filler compositions of the present invention, the biodegradable component is a sufficiently effective amount of a conventional high molecular weight hydrophilic polymer to regulate the release rate of the therapeutic agent in the void filler. Such hydrophilic polymers include hydroxyethylcellulose, hydroxypropylmethylcellulose, hydroxymethylcellulose, hydroxypropylcellulose, carboxymethylcellulose, hyaluronic acids and their salts, alginates, polyvinylpyrrolidone, polyethylene oxide, polysccarrides, chitins, hyaluronic acids, gelatin, polyacrylic acid and derivatives, gums (i.e. guar, carob bean), polymers derived from starch. These polymers can be combined with other components of the formulation by known methods such as direct mixing of powders, melt processing, or wet granulation. The bone void filler with hydrophilic polymer can be delivered to the void space in solid form, where it is exposed to physiological fluid and can hydrate into a hydrogel. The molecular weight of the hydrophilic polymer can be used to regulate the rigidity of the resulting hydrogel as well as the release rate of an active agent contained within it. Increasing molecular weight results in a decrease in the rate of release.

To extend the timed release of the therapeutic agent beyond the length of time necessary for diffusion from or erosion of hydrophilic polymer, it is possible to disperse within the hydrophilic polymer a hydrophobic degradable polymer that also contains the therapeutic agent. This can be achieved by melt-processing the hydrophobic polymer, therapeutic agent, osteoinductive component, and the hydrophilic polymer together in an extruder and placing the plug cut from the extrudate directly into the void space. Here, again, the hydration of the hydrophilic polymer into a gel is fast and dispersed within this gel matrix are domains of the hydrophobic polymer containing therapeutic agent. The therapeutic agent is partly in the hydrophilic matrix from which therapeutic agent can be released sooner and partly in the hydrophobic polymer matrix from which it is released slowly for a longer time period. In this case release rate of the therapeutic agent can be controlled by molecular weight of the hydrophilic polymer as well as the composition of the matrix (ratio of hydrophilic to hydrophobic). A sufficient amount of the hydrophobic polymer will be included in the void filler compositions to effectively provide for regulation of the rate of release of a drug or pharmaceutical agent incorporated into the void filler. The amount of hydrophilic polymer will typically be about 10 to about 70 weight percent, more typically about 15 to about 60 weight percent, and preferably about 15 to about 55 weight percent.

The bone void fillers of the present invention can be sterilized by conventional methods and processes known in the art for sterilizing biodegradable polymers with therapeutic agents.

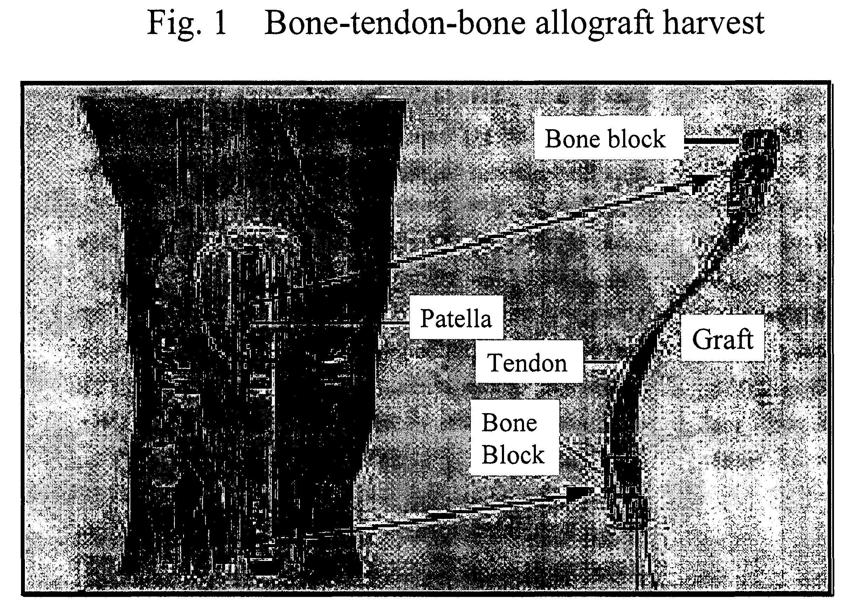

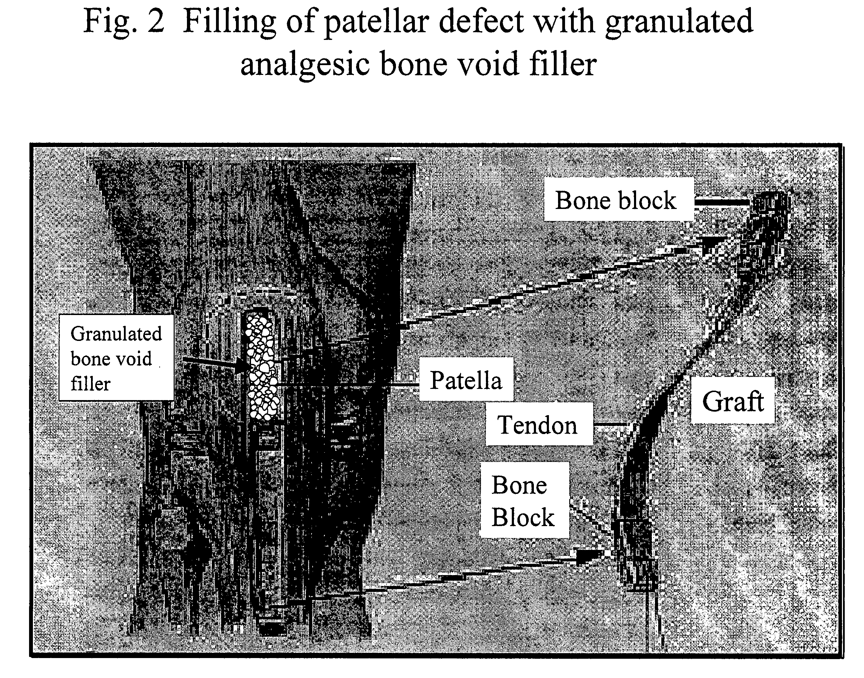

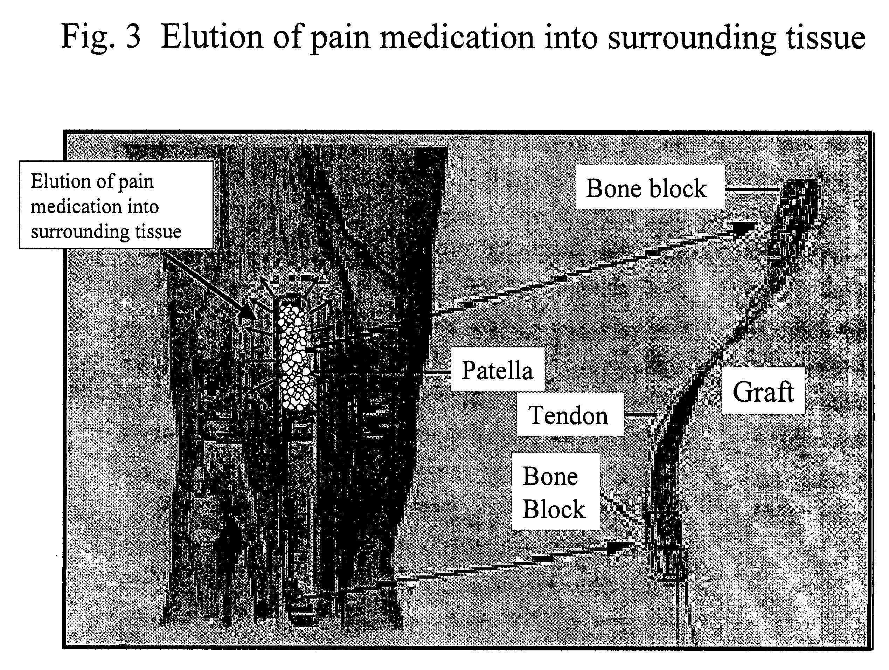

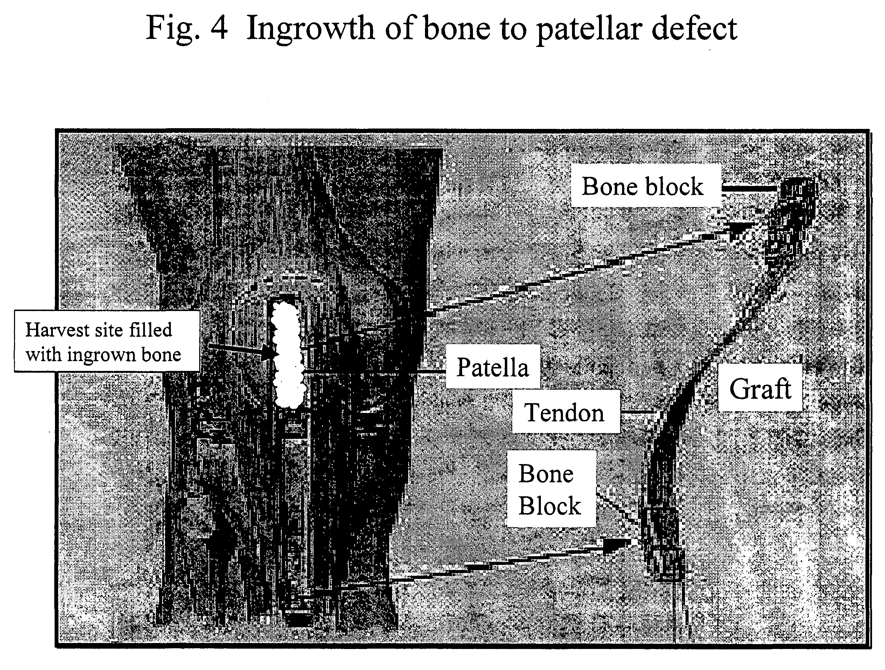

A method of using the bone void filler compositions of the present invention is illustrated in FIGS. 1-4. As seen in FIG. 1, human knee 10 is undergoing a conventional bone-tendon-bone (BTB) graft ACL reconstructive surgical procedure. The harvesting of the BTB graft 100 is illustrated. Graft 100 is seen to have patellar bone block 110, and tibial bone block 120. Bone blocks 110 and 120 are seen to be connected by patellar tendon section 130. The BTB autologous graft 100 is harvested from the patient's knee 10 using conventional surgical techniques wherein the bone block 110 is cut out from patella 20 using conventional surgical techniques and instruments, and bone block 120 is cut out from tibia 40 using conventional surgical techniques and instruments. Bone void 25 is contained in patella 20 after bone block 110 is harvested, and bone void 45 is contained in tibia 40 after bone block 130 is harvested. Patellar tendon section 130 is harvested from patellar tendon 30 resulting in opening 35 in the patellar tendon 30. Patella 20 is seen to rest upon an end 51 of femur 50. As seen in FIG. 2, granulated bone void filler composition 70 is packed into bone voids 25 and 45. The amount of bone void filler composition used may vary from substantially filling each bone void up to the top surface of the bone to using amounts that do not completely fill the bone void. Depending on the physical state of the bone void filler composition, it may be placed into bone voids in other conventional manners such as injection, extrusion, etc. After the BTB graft has been harvested and the bone void filler composition 70 of the present invention has been inserted into the bone voids 25 and 45, the ACL reconstruction procedure is completed in a conventional manner by drilling tunnels in the femur 50 and tibia 40, and then fixing the bone blocks 25 and 45 into the tunnels in femur 50 and tibia 40 respectively in a conventional manner. Alternately, the voids 25 and 45 may be filled by the void filler composition after the BTB graft has been mounted in the tunnels in the tibia 40 and femur 50. FIG. 3 illustrates the elution schematically of therapeutic agent 90 post-operatively. FIG. 4 illustrates post-operative knee 10 showing bone in-growths 27 and 47 into bone voids 25 and 45, respectively. It will be appreciated by those skilled in the art that the bone filler compositions of the present invention can be used to fill in bone voids created in a variety of additional conventional surgical procedures

The following examples are illustrative of the principles and practice of the present invention, although not limited thereto.

### Example 1: Wet Granulation Method

A granulated void filler composition of the present invention was prepared in the following manner. Hydroxyethylcellulose (HEC) (Natrosol 250HHR, Hercules, Wilmington, DE) and tricalcium phosphate (TCP) (Tri-tab, Rhodia, Cranbury, NJ) were sieved respectively through a 45 mesh screen. A 1.8-gram quantity of the sieved TCP was dry-blended with 2.0 grams of lidocaine (Sigma-Aldrich, St. Louis, MO). A 1-milliliter aliquot of isopropanol was added to the dry-blended mixture dissolving the lidocaine (Sigma-Aldrich) and suspending the TCP particles. A 1.8-gram quantity of the sieved HEC was added, in small quantities, to this mixture, blending with a spatula after each addition. Mixing was continued until appearance was uniform. The granulated mixture was transferred to an aluminum pie pan and placed on a bench top to air dry for 3 hours. Further drying occurred overnight using a vacuum oven set at 40°C. After drying the mixture was in the form of white free-flowing granules. The granules can be used as is to pack a void or they can be compressed into a precisely shaped pellet to fit a void using a tablet press.

### Example 2: Melt Processing Method

A void filler composition useful in the practice of the present invention was prepared in the following manner. Hydroxyethylcellulose (HEC) (Natrosol 250HHR; Hercules, Wilmington, DE) and tricalcium phosphate (TCP) (Tri-tab; Rhodia, Cranbury, NJ) were sieved respectively through a 45 mesh screen. A 0.5-gram quantity of sieved TCP was dry-blended with 2.0 grams of lidocaine (Sigma-Aldrich, St. Louis, MO), and 1 gram of the sieved HEC. 1.5 grams of poly(caprolactone co-dioxanone) (PCL/PDS) (Ethicon; Somerville, NJ) in the mole ratio of 95/5 was weighed out. A twin screw extruder (DACA Instruments; Goleta, CA) was heated to 85°C and half of the PCL/PDS was fed into the extruder. Polymer was allowed to melt and mix for a few minutes. The dry blend was added slowly to the extruder. Then the remaining portion of the PCL/PDS was added. The mixture was processed in the extruder for 5 minutes under a nitrogen blanket. The load initially was 500 - 600 N but reduced to approximately 300 N during processing due to the melting of the lidocaine. The extrudate emerged as a thin translucent tacky rod. Upon cooling by contact with ambient atmosphere the extrudate turned an opaque off-white in color, most likely as a result of the crystallization of the PCL. The extruded rod was brittle when cool. The extrudate rod can be cut to fit a certain size void or chopped by an impeller into small particles resembling the granules in the example above.

Alternatively, the powdered mixture can be mixed with the PCL/PDS and fabricated into a film using a compression molding process.

### Example 3: Method of use of the Bone Void Filler Composition

A patient is prepared for conventional bone-tendon-bone (BTB) graft anterior cruciate ligament (ACL) reconstructive surgery in a conventional manner. Initially, a midline incision is made from the middle of the patella to the tibial tubercle. The incision depth extends just through the paratenon of the patellar tendon. The paratenon is then reflected to expose the patellar tendon. A double-bladed knife is used to make two parallel incisions through the patellar tendon, 10 mm apart. The incisions begin at the midpoint of the patella and extend distally to a point just medial to the tibial tubercle, such that the lengths of patellar and tibial bone underneath the incision are approximately 25 mm. A sagittal saw is used to remove the bone plugs along with the section of attached patellar tendon. In this manner, approximately the middle third section of the patellar tendon is harvested, with the patellar bone block on one end and the tibial bone block on the other opposed end. The thickness of the bone plugs is typically approximately 10 mm, and results in a patellar and tibial bone defect volumes of approximately 2-3 cubic centimeters. Following ACL reconstruction using BTB autograft, the patellar bone graft site is filled with a bone void filler composition as described above. In the case of a powdered formulation, exposure to body fluids in the void results in hydration of the material, causing it to assume a putty-like consistency. After the patellar void is filled, the paratenon can be reapproximated to cover the defect. If the paratenon is not intact, the surgical site may be closed immediately after defect filling.

While the preferred embodiment of the invention applies to the repair of surgically created defects in knee surgery, there are potential applications in other medical procedures. For example, the bone void fillers of the present invention may be used in filling tooth extraction sockets in oral surgery. This would encourage quicker healing of the socket and would alleviate the substantial pain that is common to tooth extraction, especially in the mandible in which very dense, innervated cortical bone is usually found. The void fillers may also be used to fill autologous bone harvest sites in various orthopedic procedures. The iliac crest of the pelvis and the proximal tibia are common harvest sites for autologous bone for reconstructive orthopedic surgery. Oral reconstructive surgery often requires autologous bone taken from the tibial plateau, chin, mandible or roof of the mouth. Incomplete filling of such harvest sites is well known, and can leave a palpable depression in the overlying tissues. The bone void filler compositions of the present invention would promote faster healing, more complete defect filling, and alleviation of harvest site pain.

The bone void filler compositions and surgical procedures or methods of the present invention have many advantages. These advantages include elimination of excess bone defect volume at autologous graft sites, reduced likelihood of infection, alleviation of post-operative pain, and alleviation of post-operative swelling. The advantages also include reduced dependence on oral pain medications and/or external pain pumps, more rapid return to function, facilitation of physical therapy, more rapid healing and filling of bone harvest sites, more rapid mechanical reinforcement of anchor site due to enhanced bone ingrowth, controlled release of local therapeutic agents, elimination or reduction of the side effects of systemic medications, and reduction of ecchymosis from bone defect bleeding.

Although this invention has been shown and described with respect to detailed embodiments thereof, it will be understood by those skilled in the art that various changes in form and detail thereof may be made without departing from the spirit and scope of the claimed invention.

## Claims

1. A bone void filler composition, comprising:
a biodegradable material component;
an osteoconductive component; and,
a therapeutically effective amount of a therapeutic agent.

2. The composition of claim 1, wherein said biodegradable material component comprises a polymer selected from the group consisting of poly(glycolide), poly(lactide), poly(epsilon-caprolactone), poly(trimethylene carbonate), poly(para-dioxanone),and combinations thereof.

3. The composition of claim 1, wherein said biodegradable material component comprises a co-polymer selected from the group consisting of poly(lactide-co-glycolide), poly(epsilon-caprolactone-co-glycolide), poly(glycolide-co-trimethylene carbonate), and combinations thereof.

4. The composition of claim 1, wherein said biodegradable material component is selected from the group consisting of albumin, casein, waxes, starch, crosslinked starch, simple sugars, glucose, ficoll, polysucrose, polyvinyl alcohol, gelatine, modified celluloses, carboxymethylcellulose, hydroxymethyl cellulose, hydroxyethyl cellulose, hydroxypropyl cellulose, hydroxypropyl-ethyl cellulose, hydroxypropyl-methyl cellulose, sodium carboxymethyl cellulose, cellulose acetate, sodium alginate, hyaluronic acid, hyaluronic acid derivatives, polyvinyl pyrollidone, polymaleic anhydride esters, polyortho esters, polyethyleneimine, glycols, polyethylene glycol, methoxypolyethylene glycol, ethoxypolyethylene glycol, polyethylene oxide, poly(1,3 bis(p-carboxyphenoxy) propane-co-sebacic anhydride, N,N-diethylaminoacetate, block copolymers of polyoxyethylene and polyoxypropylene, and combinations thereof.

5. The composition of claim 4, wherein said biodegradable material component comprises a member selected from the group consistinf of hydroxyethyl cellulose, hyaluronic acid, and hyaluronic acid derivatives

6. The composition of claim 1 wherein said osteoconductive component is selected from the group consisting of tricalcium phosphate, alpha-tricalcium phosphate, beta-tricalcium phosphate, calcium carbonate, barium carbonate, calcium sulfate, barium sulfate, hydroxyapatite, a polymorph of calcium phosphate, and combinations thereof.

7. The composition of claim 6, wherein said osteoconductive component is beta-tricalcium phosphate.

8. The composition of claim 1, wherein said therapeutic agent is selected from the group consisting of pain medication, antiinfectives, analgesics, anti-inflammatory agents, immunosupressives, steroids, including corticosteroids, glycoproteins, lipoproteins, and combinations thereof.

9. The composition of claim 8, wherein said pain medication is selected from the group consisting of morphine, nonsteroidal anti-inflammatory drugs, oxycodone, morphine, fentanyl, hydrocodone, naproxyphene, codeine, acetaminophen with codeine, acetaminophen, benzocaine, lidocaine, procaine, bupivacaine, ropivacaine, mepivacaine, chloroprocaine, tetracaine, cocaine, etidocaine, prilocaine, procaine, clonidine, xylazine, medetomidine, dexmedetomidine, VR1 antagonists, and combinations thereof.

10. The composition of claim 8, wherein said pain medication is bupivacaine.

11. The composition of claim 1 further comprising an osteoinductive component.

12. The composition of claim 11 wherein said osteoinductive component is selected from the group consisting of cell attachment mediators, peptide- containing variations of the RGD integrin binding sequence known to affect cellular attachment, biologically active ligands, integrin binding sequence, ligands, bone morphogenic proteins, epidermal growth factor, IGF-I, IGF-II, TGF-β I-III, growth differentiation factor, parathyroid hormone, vascular endothelial growth factor, glycoprotein, lipoprotein, bFGF, TGF-β superfamily factors, BMP-2, BMP-4, BMP-6, BMP-12, BMP-14, sonic hedgehog, GDF6, GDF8, PDGF, tenascin-C, fibronectin, thromboelastin, thrombin-derived peptides, and heparin-binding domains.

13. The composition of claim 1, wherein said biodegradable material component comprises a hydrophilic polymer selected from the group consisting of hydroxyethylcellulose, hydroxypropylmethylcellulose, hydroxymethylcellulose, hydroxypropylcellulose, carboxymethylcellulose, hyaluronic acid, hyaluronic acid salts, alginates, polyvinylpyrrolidone, polyethylene oxide, polysccarrides, chitins, gelatin, polyacrylic acid, guar gum, and carob bean gum.

14. The composition of claim 1, wherein said biodegradable component comprises about 15 to about 75 weight percent.
